(19) Europäisches Patentamt

European Patent Office

Office européen des brevets

(11) **EP 1 173 283 B1**

(12) **FASCICULE DE BREVET EUROPEEN**

(45) Date de publication et mention
de la délivrance du brevet:
**06.10.2004 Bulletin 2004/41**

(51) Int Cl.⁷: **B04B 5/04**

(21) Numéro de dépôt: **00912864.6**

(22) Date de dépôt: **07.04.2000**

(86) Numéro de dépôt international:
**PCT/IB2000/000437**

(87) Numéro de publication internationale:
**WO 2000/061295 (19.10.2000 Gazette 2000/42)**

(54) **DISPOSITIF DE CENTRIFUGATION ET UTILISATION DE CE DISPOSITIF**

ZENTRIFUGATIONSVORRICHTUNG UND VERWENDUNG DIESER VORRICHTUNG

CENTRIFUGING DEVICE AND USE OF SAME

(84) Etats contractants désignés:
**DE FR GB IT**

(30) Priorité: **09.04.1999 EP 99810295**

(43) Date de publication de la demande:
**23.01.2002 Bulletin 2002/04**

(73) Titulaire: **Haemonetics Corporation
Braintree, Massachusetts 02184-9114 (US)**

(72) Inventeur: **Rochat, Jean-Denis
1272 Genolier (CH)**

(74) Mandataire: **Savoye, Jean-Paul et al
Moinas & Savoye S.A.,
42, rue Plantamour
1201 Genève (CH)**

(56) Documents cités:
**EP-A- 0 526 869**      **WO-A-88/01907**
**WO-A-88/05690**      **DE-A- 2 848 953**
**FR-A- 2 468 374**      **GB-A- 2 063 719**
**US-A- 4 109 854**      **US-A- 5 690 602**

## Description

La présente invention se rapporte à un dispositif de centrifugation de liquides notamment du sang, contenant des particules en suspension selon la revendication 1. Un tel dispositif comprend un organe de centrifugation muni d'une chambre de séparation périphérique, des moyens d'entraînement de cet organe de centrifugation en rotation autour de son axe de révolution, au moins trois canaux reliant le centre de l'organe de centrifugation à ladite chambre de séparation, les extrémités centrales de ces trois canaux étant respectivement solidaires des premières extrémités respectives de trois conduits en matériau déformable élastiquement, reliés respectivement à une source d'alimentation de liquide à centrifuger et à deux collecteurs des composants de densités différentes dudit liquide, ces conduits étant destinés à former des boucles ouvertes autour dudit organe de centrifugation orientées sensiblement radialement par rapport à son axe de rotation, les secondes extrémités respectives de ces boucles étant fixes angulairement et coaxiales à l'axe de révolution du rotor de centrifugation dont la vitesse angulaire est $2\omega$ et comprenant encore des moyens pour faire tourner chacune de ces boucles, concentriquement audit organe de centrifugation, à une vitesse angulaire $\omega$. Cette invention se rapporte également à une utilisation de ce dispositif.

Le système permettant de relier un organe tournant par un tube souple ou un cordon formant une boucle ouverte dont une extrémité est solidaire en rotation de l'axe de l'organe tournant à la vitesse $2\omega$, tandis que l'autre extrémité, coaxiale à la première, est fixe et que la boucle ouverte est entraînée à la vitesse $\omega$, engendrant une rotation du tube souple tournant autour de son propre axe à la vitesse $-\omega$, annulant ainsi toute torsion de ce tube souple, est divulgué dans le US 3'586'413. Ce principe qui permet de supprimer tout joint entre le tube souple et l'organe tournant a été largement repris dans un grand nombre de dispositifs de centrifugation. Compte tenu de la vitesse de l'organe tournant dans une centrifugeuse, le tube souple tournant sur lui-même à la vitesse $-\omega$ est soumis à une contrainte de traction engendrée par la force centrifuge, à une contrainte de flexion due à la rotation sur lui-même de la portion du tube formant la boucle ouverte à la vitesse $-\omega$, ainsi qu'à un échauffement engendré par le travail des forces visqueuses dans la matière due à la flexion susmentionnée. Or, dans le cas de la centrifugation de sang, l'élévation de température doit être < 5°C.

La prise en compte de ces différentes contraintes a conduit a des solutions utilisant des organes de centrifugation de forme relativement plate et de diamètre sensiblement supérieur à 200 mm, la vitesse de rotation $\omega$ se situant généralement aux environs de 200 rad/s, celle-ci s'élevant dans certains cas jusqu'à 400 rad/s. Ce choix permet de limiter la vitesse de rotation, de réduire la contrainte de flexion et de traction sur le tube souple tout en obtenant un débit de liquide acceptable. Ce choix qui s'est largement répandu dans le domaine des centrifugeuses pour le sang conduit évidemment à un rotor de centrifugation de diamètre relativement grand. Un tel rotor de centrifugation, compte tenu des forces centrifuges auxquelles il est soumis et de son grand diamètre doit être dimensionné pour résister à ces efforts, conduisant à un rotor de plusieurs kilos, de sorte qu'il ne devient plus possible économiquement de produire un rotor de ce type sous forme jetable, notamment lorsqu'il s'agit de plasmaphérèse dans le but de collecter du plasma.

C'est ainsi que dans le US 4'076'169 on a proposé une enceinte jetable pour la centrifugation d'un liquide contenant des particules en suspension tel que le sang. Etant donné qu'une telle enceinte jetable ne supporterait pas les efforts de centrifugation, elle est disposée dans un logement ménagé à l'intérieur d'un rotor formé de deux demi coques circulaires emboîtées l'une dans l'autre. La mise en place de l'enceinte de centrifugation dans le logement du rotor nécessite une opération de démontage et de montage du rotor, l'ouverture de celui-ci et la mise en place de l'enceinte de centrifugation.

Une solution du même type est décrite dans les US 4 010 894, dans le US 4 834 890, dans le US 4 934 995, ainsi que dans le US 4 531 932. Dans tous ces dispositifs de séparation, l'enceinte de séparation jetable est constituée par un sachet souple disposés dans un rotor de support, entraînant une manipulation beaucoup moins facile qu'avec un élément rigide.

Dans d'autres solutions, comme dans celle décrite dans le US 4'108'353, des enceintes de centrifugation sont positionnées sur le rotor présentant des éléments de positionnement et d'accrochage de ces enceintes. De nouveau, dans ce cas, la mise en place de ces enceintes requiert un certain nombre de manipulations qui sont délicates et prennent du temps. En outre un tel système requiert la présence de plusieurs enceintes, en nombre pair, pour que le rotor de centrifugation soit équilibré. Un tel système ne se prête donc pas à la centrifugation en ligne avec la prise de sang.

Les seuls rotors rigides proposés dans le cas de la séparation des constituants du sang par centrifugation sont ceux décrits dans le US 4 330 080, dans le US 4 540 397, ainsi que dans le US 5 350 514. Le rotor comporte, outre une enceinte de séparation en forme de disque de plus de 200 mm de diamètre, un corps tubulaire sur lequel sont ménagées deux surfaces annulaire de guidage et entre elles, un anneau denté destiné à venir en prise avec un pignon d'entraînement.

Le montage et le démontage de ce rotor nécessite d'écarter l'un des trois galets de guidage en prises avec chacune des surface annulaires du rotor. Etant donné que pendant la centrifugation ces galets amovibles doivent garantir le maintien du rotor, il faut prévoir des moyens de verrouillage de ces galets. Le changement de ces rotors jetables constitue donc une opération complexe et qui doit être faite avec minutie, compte tenu du danger que pourrait constituer le détachement accidentel du rotor au cours de la centrifugation.

D'autres organes de centrifugation, munis de plusieurs canaux, sont connus de GB-A-2 063 719, US-A-4 007 871 et US-A-5 690 602.

Outre ces problèmes, les dimensions et les conceptions de ces rotors conduisent à des appareils lourds, encombrants, chers qui ne sont pas adaptés à la plasmaphérèse en ligne avec la prise de sang. Si, dans une application thérapeutique, le prix d'un rotor jetable n'a pas une importance déterminante, dans le cas de la plasmaphérèse effectuée lors de collectes de sang, ce prix est déterminant. Or si les cellules sanguines, notamment les globules rouges que l'organisme met longtemps à fabriquer pouvait être réinjectées au donneur, ce donneur pourrait donner son sang à une fréquence beaucoup plus rapprochée. Or ceci n'est réalisable que si la séparation du plasma des cellules du sang peut se faire en même temps que la prise de sang et pour cela il faut avoir des enceintes de séparation qui peuvent être produites à un prix suffisamment bas et qui puissent être changées facilement et de manière fiable.

Le but de la présente invention est de remédier, au moins en partie, aux inconvénients susmentionnés.

A cet effet, cette invention a tout d'abord pour objet un dispositif de centrifugation du type susmentionné tel que défini par la revendication 1.

Elle a de plus pour objet une utilisation de ce dispositif, tel que définie par la revendication 14.

Le dispositif de centrifugation selon la présente invention permet de diviser le poids de l'organe de centrifugation d' à peu près 5 fois par rapport aux organes rigides jetables connus. Cette réduction du poids qui permet aussi d'avoir un organe de centrifugation plus compact permet de réduire le poids de l'appareil de centrifugation, de réduire son encombrement, tout en facilitant sa manipulation. Ce dispositif offre donc un nouveau champ d'application pour ce type d'appareil, puisqu'il facilite son transport du fait de sa réduction de poids et d'encombrement, outre les applications connues en milieu hospitalier, il peut être utilisé dans des véhicules de collecte du sang et permettre de réinjecter au donneur ses propres globules rouges.

D'autre avantages de ce dispositif de centrifugation apparaîtront au cours de la description qui va suivre, faite à l'aide du dessin annexé dans lequel:

la figure 1 est une vue en coupe d'une élévation de cette forme d'exécution;
la figure 2 est une vue partielle en coupe selon la ligne II-II de la figure 1,
la figure 3 est une vue schématique de la cinématique du mécanisme d'entraînement et
la figure 4 est une vue agrandie en coupe selon la ligne IV-IV de la figure 1.
la figure 5 est une vue partielle en coupe, d'une variante de la forme d'exécution de la figure 1,
la figure 6 est une vue selon la ligne VI-VI de la figure 5,
la figure 7 est une vue semblable aux figures 1 et 5 d'une autre variante.

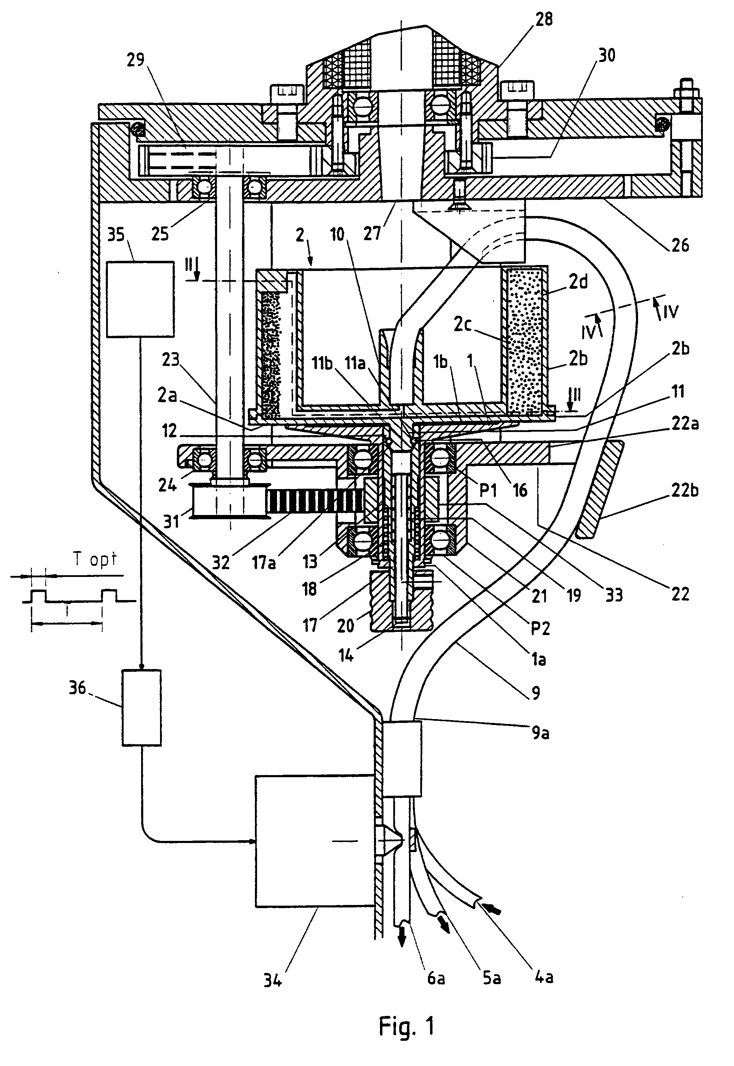

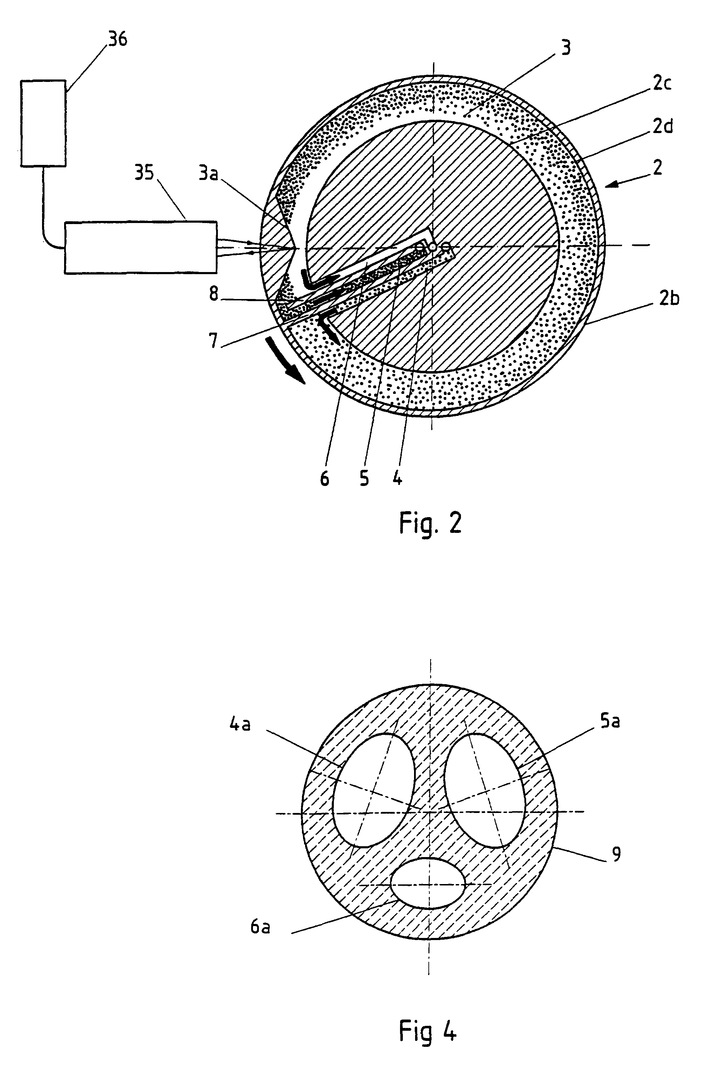

L'appareil de centrifugation illustré par la figure 1, destiné notamment à la plasmaphérèse, comporte un rotor de centrifugation, présentant la forme d'un disque 1 ménagé à l'extrémité d'un corps tubulaire 1a, monté pivotant dans deux paliers à billes P1, P2. Ce rotor de centrifugation 1 porte une cuvette de centrifugation 2 jetable, formée elle-même par la réunion de deux parties soudées ou collées l'une à l'autre, l'une inférieure, formée par un disque 2a et l'autre supérieure 2b, présentant deux parois latérales cylindriques et concentriques, l'une intérieure 2c et l'autre extérieure 2d entre lesquelles est ménagée une enceinte annulaire de séparation 3 (figures 1 et 2). Trois canaux radiaux 4, 5, 6, ménagés dans la partie supérieure 2b de la cuvette de centrifugation 2, relient cette enceinte annulaire de séparation 3 au centre de cette cuvette 2. Le canal 4 constitue le canal d'alimentation du sang à centrifuger. Il présente une cloison 7 qui rejoint la paroi latérale 2d de l'enceinte annulaire de séparation 3, tandis que l'autre paroi de ce canal d'alimentation 4 s'arrête à la paroi latérale interne 2c de cette enceinte de séparation 3.

La cloison 7 sert également à séparer le canal 4 du canal 5 destiné à la récupération des cellules du sang, dont l'autre cloison 8 s'arrête à une certaine distance de la paroi latérale externe 2d de l'enceinte annulaire de séparation 3. Cette cloison 8 sépare donc les canaux 5 et 6 et les fait communiquer respectivement avec la partie externe de l'enceinte annulaire de séparation 3, c'est-à-dire celle où se concentrent les cellules du sang, de celle de densité plus faible où se concentre le plasma. Bien évidemment, une séparation ultérieure des cellules du sang récupérées est possible pour séparer les globules rouges, les globules blancs et les plaquettes. Dans une variante de la cuvette 2, on pourrait aussi envisager d'avoir plus de deux canaux de sortie pour obtenir cette séparation.

Ces trois canaux 4, 5 et 6 aboutissent au centre de la cuvette 2 où ils sont reliés à trois conduits 4a, 5a, respectivement 6a (figure 4) qui sont, de préférence, ménagés parallèlement dans un même élément tubulaire 9 souple. La portion de cet élément tubulaire 9, adjacente à son extrémité reliée aux canaux 4, 5 et 6 est tenue dans un logement tubulaire 10 formé coaxialement à l'axe de rotation de la cuvette 2, sur la partie supérieure 2b de celle-ci. Les sections des trois conduits 4a, 5a, 6a sont elliptiques, les grands axes de ces ellipses étant tangents à au moins un cercle concentrique à l'axe longitudinal de l'élément tubulaire 9. Cette orientation des sections elliptiques des conduits 4a, 5a, 6a, facilite la rotation de l'élément tubulaire autour de son axe longitudinal.

Il ressort de ce qui précède que la partie mobile appelée à être jetée après chaque utilisation ne se compose que de trois pièces, la cuvette 2 formée de deux parties 2a, 2b soudées ou collées l'une à l'autre et l'élément tubulaire 9. En outre cet ensemble ne nécessite aucun joint d'étanchéité. Cet ensemble est relié de façon amovible au rotor de centrifugation 1 de la manière décrite ci-après.

Le fond du disque formant la partie inférieure 2a de la cuvette 2 porte un élément d'accouplement constitué par un tenon ou une tige cylindrique 11, présentant une gorge 11a de section semi-circulaire, adjacente à une extrémité tronconique 11b. Cette tige d'accouplement 11 est engagée dans un élément d'accouplement formé par une bague 12, d'un mécanisme d'accouplement 13, cette bague et ce mécanisme d'accouplement étant logés dans la partie tubulaire la du rotor 1.

Le mécanisme d'accouplement 13 comporte un moyen d'accouplement qui, dans cette forme d'exécution, est constitué par une couronne de billes 16 qui se situe à l'extrémité interne du passage axial formé par la bague 12 solidaire de la partie tubulaire la du rotor 1. Un piston tubulaire 17 est monté coulissant dans la partie tubulaire 1a. Son extrémité supérieure se termine par une surface en forme d'entonnoir 17a. Ce piston tubulaire 17 est pressé axialement contre l'extrémité interne de la bague 12 par un ressort hélicoïdal 18 comprimé entre une extrémité de la partie tubulaire la du rotor 1 et une portée du piston tubulaire 17. Cette pression axiale en direction de la bague 12 et la forme d'entonnoir 17a ont pour effet d'exercer des forces centripètes sur la couronne de billes 16 qui les presse dans la gorge 11a du tenon d'accouplement 11 de la cuvette 2.

Pour éviter que ces billes ne s'engagent dans l'ouverture axiale de la bague 12, lors de l'enlèvement du tenon d'accouplement 11, un second piston 14 est monté coulissant à l'intérieur du piston tubulaire 17 et un second ressort hélicoïdal 19 le pousse axialement contre l'extrémité de l'organe d'accouplement 11.

Selon une variante, la couronne de billes 16 pourrait être remplacée par un ressort annulaire fendu du type corde à piano, ou encore par un ressort à boudin formant un ressort torique, dont les deux extrémités seraient alors rapprochées l'une de l'autre par l'entonnoir 17a sous la pression du ressort hélicoïdal 18, réduisant ainsi son diamètre pour le maintenir en prise avec la gorge 11a du tenon d'accouplement.

L'extrémité externe du piston tubulaire 17 est solidaire d'un organe de préhension 20 destiné à permettre d'exercer une traction axiale opposée à la pression du ressort 18, pour permettre aux billes 16 de se déplacer vers l'extérieur. Le piston 14 soumis à la pression axiale du ressort 19 peut alors éjecter la cuvette 2 vers le haut et maintenir simultanément les billes 16 écartées.

Comme on peut le remarquer sur la figure 1, pour assurer un bon serrage de la cuvette 2 sur le rotor 1, la surface supérieure du disque portant cette cuvette 2 présente un léger dégagement 1b, qui assure un bon contact avec la surface annulaire périphérique de ce disque. En outre, 1a position axiale de la gorge 11a du tenon d'accouplement 11 peut être choisie pour se trouver normalement encore très partiellement dans le passage axial de la bague 12 de manière que l'engagement des billes 16 dans cette gorge 11a induise un très léger fléchissement du centre du fond de la cuvette 2, que permet le dégagement 1b du disque du rotor 1, assurant ainsi un contact suffisant entre ce disque et la cuvette 2 pour garantir un entraînement par friction de cette dernière.

Les paliers à billes P1, P2 de la partie tubulaire la du rotor sont montés dans un élément de support 21 fixé à un plateau 22, lui-même fixé à un disque supérieur 26 par quatre colonnes 15, dont deux situées derrière la cuvette 2 sont visibles sur les figures 1 et 3, les deux autres étant disposées symétriquement par rapport à un arbre d'entraînement 23 parallèle à l'axe du rotor 1. Grâce à cette disposition, le côté de l'appareil de centrifugation opposé à l'arbre d'entraînement est libre, permettant l'introduction latérale de la cuvette 2 et la mise en place de l'élément tubulaire 9. Ceci permet d'accéder aisément à la cuvette de centrifugation 2 et d'effectuer facilement sa mise en place et son enlèvement.

L'arbre d'entraînement 23 est monté pivotant par l'intermédiaire de deux roulement à billes 24, 25, respectivement solidaires du plateau 22 et du disque supérieur 26 situé au-dessus de la cuvette 2. Ce disque supérieur 26 est solidaire de l'arbre d'entraînement 27 d'un moteur 28, coaxial à l'axe de rotation du rotor 1. L'extrémité de l'arbre 23 qui s'étend au-dessus du disque 26 est solidaire d'un pignon satellite 29 en prise avec un pignon fixe 30. Le rapport entre les diamètres du pignon satellite 29 et du pignon fixe 30 est de 1/1, de sorte que si la vitesse de rotation du plateau 26 est $\omega$, celle de l'arbre 23 autour de son axe est $2\omega$. L'extrémité inférieure de cet arbre 23 porte un pignon cranté 31 relié par une courroie crantée 32 à un pignon cranté 33, de même diamètre que le pignon cranté 31, de sorte que le rotor 1 est entraîné à la vitesse $2\omega$.

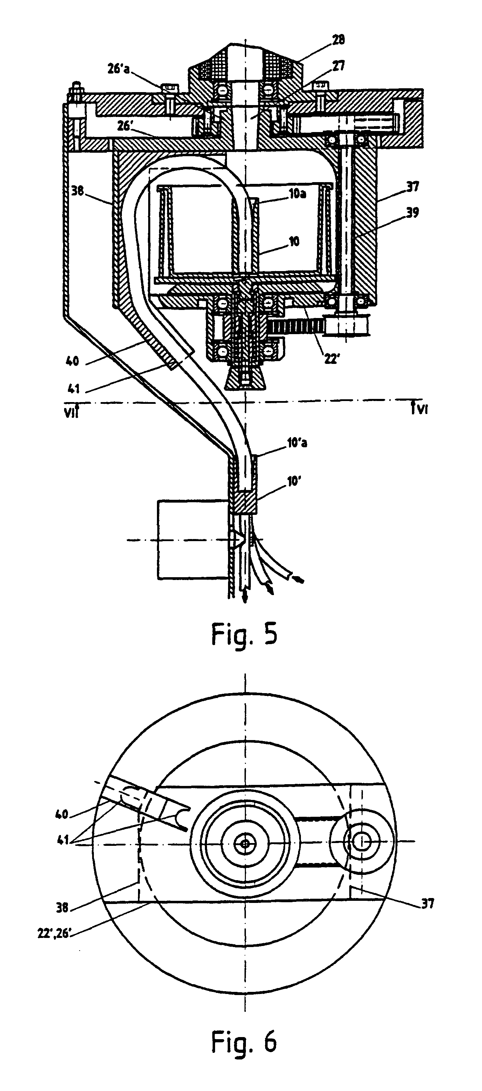

L'élément tubulaire souple 9 forme une boucle ouverte dont une extrémité 9a est fixe et coaxiale à l'axe de pivotement du rotor 1. Cette extrémité 9a est fixée et tenue dans un logement tubulaire de raccord 10' similaire au logement 10 supportant l'autre extrémité de cet élément tubulaire 9. Chacun de ces éléments tubulaire 10 et 10' présente une sorte d'entonnoir 10a, respectivement 10'a (fig. 5) qui confère un appui à cette partie de l'élément tubulaire 9 lorsqu'il est soumis à la force centrifuge. Cette boucle passe à travers une ouverture 22a ménagée dans le plateau 22, de sorte qu'elle est entraînée autour de l'axe de pivotement du rotor 1 à la vitesse $\omega$, alors que son extrémité solidaire du centre de la cuvette 2 est entraînée à la vitesse $2\omega$ et que l'autre extrémité 9a est fixe, de sorte que l'élément souple est entraîné entre ces deux extrémités à la vitesse -$\omega$ autour de son axe longitudinal annulant toute accumulation de torsion entre ces deux extrémités. Ce principe est bien connu depuis le US 3 586 413 de Adams. Une surface d'appui 22b solidaire du plateau 22 sert à limiter la déformation de l'élément tubulaire 9 sous l'effet de la force centrifuge. Les parties de guidage de l'élément tubulaire 9 sont de préférence en un matériau autolubrifiant ou à faible coefficient de frottement, tel que Oilamid®, bronze-Teflon®, ou Valflon®.

En aval de l'extrémité fixe 9a de l'élément tubulaire 9, les trois conduits 4a, 5a, 6a se séparent et le conduit de plasma 6a est associé à une valve de réglage 34 du débit en fonction de la position de la surface de séparation entre le plasma et les cellules du sang dans l'enceinte de séparation 3.

A cet effet, un double prisme 3a est ménagé à l'extrémité supérieure de l'enceinte de séparation 3 et est venu d'une pièce avec la partie supérieure 2b de la cuvette 2 lors de son injection. La portion de ce double prisme 3a qui est recouverte par les cellules du sang qui ont été séparées du plasma par la force centrifuge consécutive à la rotation de la cuvette 2 est opaque, tandis que la partie qui émerge dans le plasma est transparente. Un dispositif optique 35 comprenant un laser et un détecteur photoélectrique est disposé vis-à-vis de ce prisme 3a, de sorte que le détecteur photoélectrique reçoit la lumière réfléchie par la partie du double prisme 3a qui émerge dans le plasma transparent. A chaque tour de la cuvette 2, un signal de durée proportionnelle à la valeur angulaire de la zone transparente du double prisme 3a est ainsi fourni à un amplificateur 36 dont la sortie est reliée à la valve proportionnelle 34. Suivant que cette zone transparente augmente ou diminue, l'amplificateur 36 commandera la valve proportionnelle 34 pour qu'elle réduise, respectivement pour qu'elle augmente la section du conduit 6a d'évacuation du plasma, permettant de maintenir par ce réglage, l'équilibre entre les écoulements dans les conduits de sortie 5a et 6a, ceci en fonction d'un débit d'entrée déterminé par la pompe d'alimentation du sang dans le conduit 4a, elle-même déterminée par la pression veineuse du bras du donneur.

Le dimensionnement de la cuvette de centrifugation 2 et de l'élément tubulaire 9 formant la boucle ouverte sont choisis pour permettre de réduire l'encombrement, le poids, le prix et le volume, aussi bien de cette cuvette 2 que de l'ensemble de l'appareil de centrifugation dont le dimensionnement est essentiellement tributaire du diamètre de la cuvette de centrifugation. Si le diamètre diminue, il est nécessaire d'augmenter la vitesse. L'augmentation de celle-ci peut être limitée par l'augmentation de la hauteur de l'enceinte de centrifugation 3, pour que le débit maximum résultant reste pratiquement constant, celui-ci étant déterminé par l'obtention d'une bonne sédimentation des cellules sanguines.

A titre d'exemple le diamètre de la cuvette est de 80 mm et sa hauteur est sensiblement égale à son rayon. Un tel diamètre correspond environ au tiers de celui des rotors de séparation de l'état de la technique. Par conséquent, la longueur de la boucle ouverte formée par le conduit tubulaire 9 correspond donc sensiblement au tiers des boucles de l'état de la technique.

En réduisant le rayon de la cuvette 2 et ainsi la longueur de la boucle formée par le conduit tubulaire 9, la force de traction exercée sur elle par la force centrifuge à laquelle elle est soumise peut être maintenue à une valeur constante. Au lieu d'utiliser trois tuyaux de 4 mm de diamètre nous avons un seul élément tubulaire 9 de 7 mm de diamètre, de sorte que la section résultante est la même, soit 0,38 cm$^2$. Le matériau de l'élément tubulaire est du PVC plastifié ou du silicone dont le poids spécifique est 1,2 g/cm$^3$, comme dans l'état de la technique. Etant donné que la longueur de la boucle ouverte de l'élément tubulaire 9 est réduite au tiers de celle de l'état de la technique, la masse de cet élément tubulaire correspond donc également sensiblement au tiers. Le rayon de la boucle ouverte est aussi réduit pratiquement au tiers.

La force de traction F exercée sur ce tuyau correspond à:

$$F = m\omega^2.R$$

Dans l'état de la technique on obtient avec une vitesse de la boucle de 1000 t/min ($\omega \cong 100$) correspondant à la moitié de la vitesse du rotor qui est de 2000 t/min et avec un rayon de boucle de 0,13 m, une force de:

$$F = 0,014.100^2.0,13 = 18,2N$$

Dans le cas de l'exemple selon la présente invention, avec une masse de 0,0046 Kg, une vitesse de la boucle de 3000 t/min (correspondant à une vitesse du rotor 1 de 6000 t/min) et un rayon de boucle de 0,045 m, la force est de:

$$F = 0,0046.300^2.0,045 = 18,6 \text{ N}$$

La valeur des efforts de traction est:

$$\sigma = F/S = 18/38 = 0,47 \text{ N/mm}^2$$

Etant donné que la valeur des efforts des flexions alternées sur l'élément tubulaire correspond à:

$$\sigma = E.r/R$$

où r, rayon de l'élément tubulaire
et R, rayon de la boucle formée par cet élément tubulaire.

Le rayon R étant plus petit dans le cas de la présente invention, pour diminuer σ, il faut donc diminuer r et E. Dans l'exemple donné, E = 4 N/mm$^2$ et

$\sigma_{rupture}$ = 12 N/mm$^2$. Dans le cas de flexions correspondant à 1 million de flexions alternées, soit 5 ½ heures de fonctionnement, cette valeur est réduite d'un facteur de 5 pour tenir compte de la fatigue supplémentaire, de sorte que $\sigma_{rupture}$ pour un effort de flexion alterné = 2,4 N/mm$^2$.

$$\sigma = 4.3,5/30 = 0,47 \text{ N/mm}^2$$

soit un coefficient de sécurité de 2,4/0,47 $\cong$ 5

Cet exemple de dimensionnement montre qu'il est tout à fait possible de réduire très sensiblement le diamètre de l'enceinte de séparation sans perte de performances et sans augmentation des contraintes pour autant que certaines mesures soient prises à cet effet. Or, cette réduction de diamètre permet de réduire la taille de l'appareil de manière extrêmement importante. Ceci permet d'avoir un appareil beaucoup plus compact, plus léger et moins coûteux à fabriquer. Cet appareil prenant peu de place, on peut installer davantage d'appareils sur une même surface, ce qui est important, notamment dans le cas de camions utilisés pour la collecte de plasma où la place est réduite.

A titre d'exemple, la partie en rotation selon l'invention pèse environ 600 g alors que les rotors des appareils de l'état de la technique pèsent pratiquement cinq fois ce poids. C'est la raison pour laquelle dans la collecte de sang, la plasmaphérèse n'est généralement pas effectuée directement, mais que le sang est récolté dans des poches souples qui sont ensuite placées dans de très grosses centrifugeuses. Dans ce cas, il n'est plus possible de rendre au donneur ses globules rouges. Or, le temps pour que l'organisme reproduise la quantité de globules rouges est long, ce qui explique pourquoi plusieurs mois doivent nécessairement séparer deux dons du sang pour un même donneur, ce qui ne serait pas nécessaire si les globules rouges pouvaient lui être réinjectée après séparation. Or ceci n'est possible que si la séparation est faite simultanément à la prise de sang.

Il existe d'autres types de machines fonctionnant avec un bol de centrifugation à usage unique, mais ceux-ci nécessitent un joint tournant, conduisant à une solution plus chère et ne permettant pas d'effectuer simultanément l'alimentation du liquide à centrifuger et l'évacuation des composants séparés, de sorte qu'il est nécessaire d'alterner l'alimentation et l'évacuation, conduisant à un gros volume extracorporel.

L'importance d'avoir des appareils de centrifugation légers et de faible encombrement et surtout des enceintes de séparation jetables pouvant être produites à bas prix est donc évidente. La facilité de changement de ces enceintes ou cuvettes de séparation est par conséquent également une nécessité. Seule la réunion de l'ensemble de ces conditions peut permettre de remplacer les méthodes actuelles de collecte du plasma.

Un autre aspect important de cette invention réside dans le fait que la circulation complète du liquide est obtenue par la surpression avec laquelle le sang est amené dans la cuvette de centrifugation 2. Cette surpression doit compenser les pertes de charges induites dans le conduit d'alimentation 4a ainsi que dans les conduits de récupération des cellules du sang 5a et du plasma 6a. Pour créer cette surpression on peut avantageusement utiliser une pompe péristaltique, destinée à assurer le débit entrant désiré. Aucune pompe d'aspiration péristaltique des composants sortants n'est donc nécessaire, la régulation du débit de plasma étant obtenue par la valve de réglage 34, commandée par son système d'asservissement en fonction de la variation de position de la frontière entre le plasma et les cellules du sang.

Bien entendu, si cet appareil est apte en particulier à une utilisation pour effectuer la plasmaphérèse en ligne avec la prise de sang, il peut bien entendu également être utilisé dans des applications à caractère thérapeutique. On a pu constater en effet que l'élément tubulaire 9 renfermant les trois conduits 4a, 5a, 6a est calculé avec un facteur de sécurité de 5 pour une utilisation continue dépassant 5 heures, ce qui permet son utilisations dans toutes les applications envisageables.

L'appareil objet de la présente invention peut aussi être utilisé pour le lavage de cellules sanguines en introduisant alternativement avec des moyens adaptés connus dans le domaine, les cellules à laver et un liquide de lavage. En variante, le liquide de lavage pourrait être introduit par un conduit supplémentaire, permettant d'effectuer simultanément la séparation et le lavage. Dans ce cas, l'élément tubulaire 9 devrait alors comporter quatre conduits au lieu des trois représentés.

Dans la variante illustrée par les figures 5 et 6, les deux disques 22 et 26 de la forme d'exécution précédente sont remplacés par deux bras diamétraux 22', 26' qui sont venus d'une seule pièce en aluminium avec deux piliers 37 et 38 diamétralement opposés. Le bras 26' présente un moyeu 26'a qui est chassé sur l'arbre 27 du moteur 28. Le pilier 37 présente un passage cylindrique 39 destiné au passage de l'arbre d'entraînement 23. L'autre pilier 38 est solidaire d'un support 40 présentant une goulotte de guidage 41 de l'élément tubulaire 9.

Le support 40 est conçu pour soutenir l'élément tubulaire souple 9 dans la zone où son rayon est le plus grand, donc où la force centrifuge est la plus importante. l'entonnoir 10a lui, soutient la partie centrale de l'élément tubulaire 9.

Pour réduire le frottement entre la goulotte 41 du support 40 et l'élément tubulaire 9 pendant la rotation de l'appareil, le support 40 est fait, comme le support 22b de la forme d'exécution de la figure 1, en un matériau à faible coefficient de frottement. Outre les matériaux déjà cités, on pourrait aussi utiliser un polyéthylène à haut poids moléculaire (PEHMW). On peut encore favoriser le glissement en utilisant lors de la fabrication de l'élément tubulaire 9, lorsque celui-ci est en PVC, un plastifiant à base de silice rendant sa surface plus glissante. Il est encore possible de réduire le frottement en diminuant la surface de contact de la goulotte 41 par des stries éventuellement en forme de vrilles.

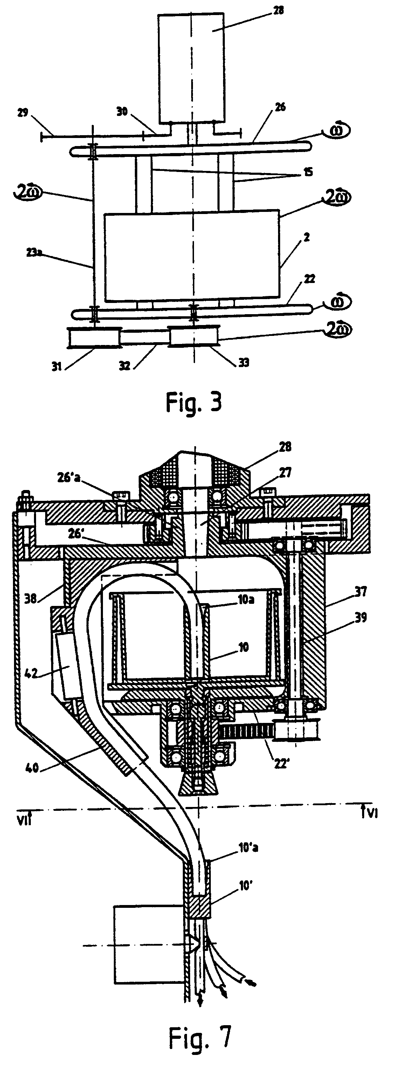

Selon une dernière variante illustrée par la figure 7, on dispose dans la goulotte du support 40 des rouleaux 42 pivotés librement autour d'axes parallèles à celui de l'élément tubulaire 9. Ces rouleaux 42 sont entraînés par la rotation de l'élément tubulaire 9 sur lui-même.

Le reste de l'appareil de centrifugation correspond à la forme d'exécution décrite précédemment. La variante décrite en relation avec les figures 5 et 6 facilite l'équilibrage et permet d'augmenter la sécurité de l'appareil lorsqu'il tourne à la vitesse de centrifugation. Elle améliore également le guidage et le soutien de l'élément tubulaire 9, qui est ainsi très peu soumis à la force centrifuge.

**Revendications**

**1.** Dispositif de centrifugation de liquides contenant des particules en suspension notamment du sang, comprenant un organe de centrifugation amovible (2) présentant un axe de rotation, une chambre de séparation périphérique (3), une pluralité de canaux (4, 5, 6) reliant le centre de cet organe de centrifugation (2) à ladite chambre de séparation (3), les extrémités centrales de ces canaux (4, 5, 6) étant respectivement solidaires des premières extrémités respectives de conduits (4a, 5a, 6a), les secondes extrémités respectives de ces conduits étant fixes angulairement et coaxiales audit axe de rotation; des premiers moyens d'entraînement (22) pour faire tourner la pluralité de conduits autour dudit axe de rotation, à une vitesse angulaire ω, et des seconds moyens d'entraînement (1, 23-33) pour faire tourner cet organe de centrifugation (2) en rotation autour dudit axe de rotation à une vitesse angulaire 2ω, **caractérisé en ce que** ledit organe de centrifugation (2) a un rayon compris entre 25-50 mm, et une hauteur comprise entre 75-125% de ce rayon.

**2.** Dispositif de centrifugation selon la revendication 1, **caractérisé en ce que** de l'organe de centrifugation (2) est apte à tourner à une vitesse angulaire 2ω > 500 rad/s.

**3.** Dispositif de centrifugation, selon la revendication 1, **caractérisé en ce que** le liquide introduit dans l'organe de centrifugation (2) est apte à s'écouler à un débit d'au moins 100 ml/min.

**4.** Dispositif de centrifugation selon la revendication 1, **caractérisé en ce que** lesdits conduits (4a, 5a, 6a) sont aptes à résister à une force de traction < 0,7 N/mm², que leur module d'élasticité est < 5 N/mm², et que leur résistance à la rupture à la flexion alternée est supérieure à 1,5 N/mm².

**5.** Dispositif selon la revendication 1, **caractérisé en ce que** ledit organe de centrifugation (2) à la forme d'une cuvette présentant deux parties circulaires conformées pour ménager entre elles lesdits canaux (4, 5, 6) et ladite chambre de séparation (3).

**6.** Dispositif selon l'une des revendications précédentes, **caractérisé en ce que** les trois conduits (4a, 5a, 6a) sont. intégrés dans un même élément tubulaire (9).

**7.** Dispositif selon l'une des revendications précédentes, **caractérisé en ce que** le diamètre de la section dudit élément tubulaire (9) n'excède pas 7 mm.

**8.** Dispositif selon la revendication 7, **caractérisé en ce que** les sections des trois conduits (4a, 5a, 6a) sont elliptiques, les grands axes de ces ellipses étant tangents à au moins un cercle concentrique à l'axe longitudinal dudit élément tubulaire (9).

**9.** Dispositif selon l'une des revendications précédentes, **caractérisé en ce qu'**il comporte des moyens de support (22b, 40) pour limiter la déformation desdites boucles formées par l'élément tubulaire souple (9) sous l'effet de la force centrifuge.

**10.** Dispositif selon l'une des revendications précédentes, **caractérisé en ce qu'**un (6a) desdits conduits destiné à être relié à un collecteur de l'un des composants issu de la centrifugation comporte une valve proportionnelle (34), des moyens de détection étant disposé en amont de ce conduit pour mesurer le degré de pureté du composant devant s'écouler par ledit conduit, ce détecteur étant relié à ladite valve proportionnelle (34) pour régler le débit dans ledit conduit (6a) en fonction dudit degré de pureté mesuré.

**11.** Dispositif selon la revendication 1, **caractérisé en ce qu'**un espace est ménagé latéralement aux premier et second moyens d'entraînement (22, 1) pour permettre le passage dudit organe de centrifugation.

**12.** Dispositif selon la revendication 10, **caractérisé en ce qu'**un prisme est disposé dans ladite chambre de séparation (3), lesdits moyens de détection comportant un faisceau laser fixe par rapport audit organe de centrifugation (2) et une cellule photoélectrique pour mesurer la position de la frontière entre les cellules du sang et le plasma et' délivrer à un organe de commande de ladite valve proportionnelle, un signal caractéristique de cette position.

**13.** Dispositif selon l'une des revendications précédentes, **caractérisé en ce que** le frottement entre l'élément tubulaire (9) et les parties mobiles en contact avec lui est réduit par l'incorporation en surface d'un plastifiant à base de silice sur PVC de cet élément tubulaire (9) et/ou par une réduction de la surface de contact de la goulotte (22b, 41) par des stries en forme de vrilles.

**14.** Utilisation du dispositif selon l'une des revendications précédentes, **caractérisé en ce qu'**on met ledit liquide à centrifuger sous une pression (P) apte à produire un écoulement à débit déterminé, on mesure le degré de pureté d'au moins un des composants centrifugés et on règle la proportion des débits respectifs des deux composants issus de la centrifugation, en fonction dudit degré de pureté.

**Claims**

**1.** Device for the centrifugation of liquids containing particles in suspension, especially for the centrifugation of blood, comprising a removable centrifugation member (2) having a rotation axis, a peripheral separating chamber (3), a plurality of channels (4, 5, 6) connecting the centre of this centrifugation member (2) to the said separating chamber (3), the central ends of these channels (4, 5, 6) being respectively fastened to the first respective ends of pipes (4a, 5a, 6a), the second respective ends of these pipes being angularly fixed and coaxial with the said axis of rotation; first drive means (22) for rotating the plurality of pipes around the said axis of rotation at an angular velocity $\omega$, and second drive means (1, 23 - 33) for rotating this centrifugation member (2) so as to rotate about the said axis of rotation at an angular velocity $2\omega$, **characterized in that** the said centrifugation member (2) has a radius of between 25 and 50 mm and a height of between 75 and 125% of this radius.

**2.** Centrifugation device according to Claim 1, **characterized in that** the centrifugation member (2) is capable of rotating at an angular velocity $2\omega$ > 500 rad/s.

**3.** Centrifugation device according to Claim 1, **characterized in that** the liquid introduced into the centrifugation member (2) is capable of flowing at a rate of at least 100 ml/min.

**4.** Centrifugation device according to Claim 1, **characterized in that** the said pipes (4a, 5a, 6a) are capable of withstanding a tensile force < 0.7 N/mm$^2$, **in that** their elastic modulus is < 5 N/mm$^2$ and **in that** their flexural strength in reverse bending is greater than 1.5 N/mm$^2$.

**5.** Device according to Claim 1, **characterized in that** the said centrifugation member (2) is in the form of a bowl having two circular parts shaped so as to leave between them the said channels (4, 5, 6) and the said separating chamber (3).

**6.** Device according to one of the preceding claims, **characterized in that** the three pipes (4a, 5a, 6a) are integrated into one and the same tubular element (9).

**7.** Device according to one of the preceding claims, **characterized in that** the diameter of the cross section of the said tubular element (9) does not exceed 7 mm.

**8.** Device according to Claim 7, **characterized in that** the cross sections of the three pipes (4a, 5a, 6a) are elliptical, the major axes of these ellipses being tangents to at least one circle concentric with the longitudinal axis of the said tubular element (9).

**9.** Device according to one of the preceding claims, **characterized in that** it includes support means (22b, 40) for limiting the deformation of the said loops formed by the flexible tubular element (9) under the effect of the centrifugal force.

**10.** Device according to one of the preceding claims, **characterized in that** one (6a) of the said pipes, which is intended to be connected to a collector of one of the components resulting from the centrifugation, includes a proportional valve (34), detection means being placed upstream of this pipe in order to measure the degree of purity of that component which has to flow out via the said pipe, this detector being connected to the said proportional valve (34) in order to adjust the flow rate in the said pipe (6a) according to the said degree of purity measured.

**11.** Device according to Claim 1, **characterized in that** a space is provided, laterally to the first and second drive means (22, 1), in order to allow the said centrifugation member to pass through.

**12.** Device according to Claim 10, **characterized in that** a prism is placed in the said separating chamber (3), the said detection means comprising a laser beam fixed relative to the said centrifugation member (2) and a photoelectric cell in order to measure the position of the boundary between the blood cells and the plasma and to deliver, to a member for controlling the said proportional valve, a signal characteristic of this position.

**13.** Device according to one of the preceding claims, **characterized in that** the friction between the tubular element (9) and the moving parts in contact with the latter is reduced by the incorporation, on the surface, of a plasticizer based on silica on this PVC tubular element (9) and/or by a reduction in the area of contact of the chute (22b, 41) by serration in the form of kinks.

14. Use of the device according to one of the preceding claims, **characterized in that** the said liquid to be centrifuged is put under a pressure (P) capable of producing a flow at a defined flow rate, the degree of purity of at least one of the centrifuged components is measured and the proportion of the respective flow rates of the two components resulting from the centrifugation are adjusted according to the said degree of purity.


**Patentansprüche**

1. Zentrifugiervorrichtung für Flüssigkeiten und insbesondere Blut, die suspendierte Teilchen enthalten, mit einem abnehmbaren Zentrifugierorgan (2), das eine Drehachse und eine periphere Trennkammer (3) aufweist, mit mehreren Kanälen (4, 5, 6), die die Mitte dieses Zentrifugierorgans (2) mit der Trennkammer (3) verbinden, wobei die zentralen Enden dieser Kanäle (4, 5, 6) jeweils mit den ersten Enden von Röhren (4a, 5a, 6a) fest verbunden sind und die zweiten Enden dieser Röhren in ihrer Winkellage feststehend und koaxial zu der benannten Drehachse sind; mit ersten Antriebsmitteln (22), um die Mehrzahl von Röhren mit einer Winkelgeschwindigkeit ω um die benannte Drehachse drehen zu lassen; sowie mit zweiten Antriebsmitteln (1, 23 - 33), um dieses Zentrifugierorgan (2) mit einer Winkelgeschwindigkeit 2ω in Drehung um die benannte Drehachse zu versetzen, **dadurch gekennzeichnet, dass** das Zentrifugierorgan (2) einen Radius zwischen 25 und 50 mm und eine Höhe zwischen 75 und 125 % dieses Radius besitzt.

2. Zentrifugiervorrichtung nach Anspruch 1, **dadurch gekennzeichnet, dass** das Zentrifugierorgan (2) in der Lage ist, mit einer Winkelgeschwindigkeit 2ω von mehr als 500 rad/s zu rotieren.

3. Zentrifugiervorrichtung nach Anspruch 1, **dadurch gekennzeichnet, dass** die in das Zentrifugierorgan (2) eingeführte Flüssigkeit mit einer Strömungsgeschwindigkeit von wenigstens 100 ml/min fliessen kann.

4. Zentrifugiervorrichtung nach Anspruch 1, **dadurch gekennzeichnet, dass** die benannten Röhren (4a, 5a, 6a) in der Lage sind, einer Zugkraft von <0,7 N/mm$^2$ zu widerstehen, dass ihr Elastizitätsmodul <5 N/mm$^2$ beträgt und dass ihr Bruchwiderstand gegenüber Biegewechselbeanspruchung mehr als

1,5 N/mm$^2$ beträgt.

5. Vorrichtung nach Anspruch 1, **dadurch gekennzeichnet, dass** das benannte Zentrifugierorgan (2) die Gestalt einer Schale hat, die zwei kreisrunde Teile aufweist, die so geformt sind, dass sie zwischen sich die benannten Kanäle (4, 5, 6) und die benannte Trennkammer (3) aufnehmen.

6. Vorrichtung nach einem der vorangehenden Ansprüche, **dadurch gekennzeichnet, dass** die drei Röhren (4a, 5a, 6a) in ein und dasselbe röhrenförmige Element (9) integriert sind.

7. Vorrichtung nach einem der vorangehenden Ansprüche, **dadurch gekennzeichnet, dass** der Durchmesser des Querschnitts des röhrenförmigen Elements (9) 7 mm nicht übersteigt.

8. Vorrichtung nach Anspruch 7, **dadurch gekennzeichnet, dass** die Querschnitte der drei Röhren (4a, 5a, 6a) elliptisch sind, wobei die grossen Achsen dieser Ellipsen zu zumindest einem zur Längsachse des röhrenförmigen Elements (9) konzentrischen Kreis tangential sind.

9. Vorrichtung nach einem der vorangehenden Ansprüche, **dadurch gekennzeichnet, dass** sie Stützorgane (22b, 40) umfasst, um die Verformung der durch das weiche röhrenförmige Element (9) gebildeten Schleifen unter der Einwirkung der Zentrifugalkraft zu begrenzen.

10. Vorrichtung nach einem der vorangehenden Ansprüche, **dadurch gekennzeichnet, dass** eine (6a) der benannten Röhren, die dazu bestimmt ist, an einen Sammler einer der durch das Zentrifugieren erzeugten Komponenten angeschlossen zu werden, ein Proportionalventil (34) umfasst, wobei Detektionsorgane strömungsaufwärts von dieser Röhre angeordnet sind, um den Reinheitsgrad der Komponente zu messen, die durch diese Röhre ausströmen soll, und dieser Detektor an das Proportionalventil (34) angeschlossen ist, um die Durchflussmenge in dieser Röhre (6a) in Abhängigkeit von dem gemessenen Reinheitsgrad zu regeln.

11. Vorrichtung nach Anspruch 1, **dadurch gekennzeichnet, dass** seitlich vom ersten und zweiten Antriebsorgan (22, 1) Raum freigelassen ist, um den Durchgang des Zentrifugierorgans zu ermöglichen.

12. Vorrichtung nach Anspruch 10, **dadurch gekennzeichnet, dass** ein Prisma in der Trennkammer (3) angeordnet ist, wobei die benannten Detektionsmittel einen Laserstrahl, der bezüglich des Zentrifugierorgans (2) stationär ist, sowie eine photoelektrische Zelle umfassen, um die Position der Grenze zwischen den Blutkörperchen und dem Plasma zu messen und einem Steuerorgan des Proportionalventils ein diese Position charakterisierendes Signal zu liefern.

13. Vorrichtung nach einem der vorangehenden Ansprüche, **dadurch gekennzeichnet, dass** die Reibung zwischen dem röhrenförmigen Element (9) und den mit ihm in Berührung stehenden beweglichen Teilen durch Einarbeitung eines Weichmachers auf der Basis von Siliciumdioxid in die Oberfläche des PVC dieses röhrenförmigen Elements (9) und/oder durch eine Verkleinerung der Berührungsfläche der Rinne (22b, 41) durch schraubenförmige Streifen verringert wird.

14. Verwendung der Vorrichtung nach einem der vorangehenden Ansprüche, **dadurch gekennzeichnet, dass** die zu zentrifugierende Flüssigkeit unter einen Druck (P) gesetzt wird, der in der Lage ist, eine bestimmte Strömungsgeschwindigkeit zu erzeugen, dass der Reinheitsgrad von zumindest einer der zentrifugierten Komponenten gemessen wird und dass das Verhältnis der Strömungsgeschwindigkeiten der beiden aus dem Zentrifugieren hervorgehenden Komponenten in Abhängigkeit von dem benannten Reinheitsgrad geregelt wird.

Fig. 1

Fig. 2

Fig 4

Fig. 5

Fig. 6

13

Fig. 3

Fig. 7